# FASCICULE DE BREVET EUROPEEN

(11) **EP 2 477 595 B1**
(45) Date de publication et mention de la délivrance du brevet: **22.07.2015**
(21) Numéro de dépôt: 10763445.3
(22) Date de dépôt: 17.09.2010
(51) Int. Cl.: A61K 8/33, A61K 8/40, C07C 47/11, C07C 47/225, C07C 255/03, C07C 255/07, C11B 9/00, C07C 253/30, C07C 255/31

(54) **NOUVEAUX ALDEHYDES ET NITRILES ISSUS DE L'ISOPHORONE, AINSI QUE LEUR UTILISATION EN PARFUMERIE**
NEUE ALDEHYDE UND NITRILE AUS ISOPHORON SOWIE VERWENDUNG DAVON IN DER PARFÜMERZEUGUNG
NOVEL ALDEHYDES AND NITRILES FROM ISOPHORONE, AND THE USE THEREOF IN PERFUMERY

(30) Priorité: 18.09.2009 FR 0956422
(43) Date de publication de la demande: 25.07.2012
(73) Titulaire: V. Mane Fils, 06620 Bar sur Loup (FR)
(72) Inventeur: MURATORE, Agnès, 06740 Chateauneuf (FR); CHANOT, Jean-Jacques, F-06530 Speracedes (FR)
(74) Mandataire: Dias, Sonia
(86) Numéro de dépôt international: PCT/IB2010/054213
(87) Numéro de publication internationale: WO 2011/033479

(56) Documents cités:
- US-A- 4 477 683
- US-A- 5 073 538

## Description

La présente invention concerne de nouveaux aldéhydes et nitriles issus de l'isophorone présentant une fragrance particulière ainsi que l'utilisation de ces composés en parfumerie.

Le terme « parfumerie » est ici utilisé pour désigner non seulement la parfumerie au sens habituel du terme, mais également les autres domaines dans lesquels l'odeur des produits est importante. Il peut s'agir de compositions de parfumerie au sens habituel du terme, telles que bases et concentrés parfumants, eaux de Cologne, eaux de toilette, parfums et produits similaires ; de compositions topiques - en particulier cosmétiques - telles que crèmes pour le visage et le corps, poudres de talc, huiles pour cheveux, shampoings, lotions capillaires, sels et huiles de bain, gels de douche et de bain, savons de toilette, antitranspirants et désodorisants corporels, lotions et crèmes de rasage, savons, crèmes, dentifrices, bains de bouche, pommades, et produits similaires ; et de produits d'entretien, tels qu'assouplissants, détergents, lessives, désodorisants d'ambiance, et produits similaires.

Les termes « fragrance », « fragrant », « odorant » sont utilisés ici de manière interchangeable pour tout composé organoleptique stimulant de façon plaisante l'odorat.

Par le terme « masquer » ou « masquant » on entend réduire ou éliminer la perception d'une mauvaise odeur ou d'un mauvais goût générée par une ou plusieurs molécules entrant dans la composition d'un produit.

De nombreux dérivés de type cyclopentanique, et en particulier les composés comportant un squelette campholénique ou les cyclopentanones, sont décrits dans la littérature pour leurs propriétés olfactives intéressantes. Par exemple, les demandes de brevet US 5073538, EP 0146859, US 4477683 et Bruns et al. (Bruns K. ; Weber U. ; Development in food science, 1998, 18 (F&F), 767-776) décrivent des dérivés du 2,4,4-triméthylcyclopentane dont certains présentent un intérêt pour l'industrie de la parfumerie. Cependant, très peu de composés de type nitriles ou aldéhydes dérivés du 2,4,4-triméthylcyclopentane ont été décrits dans la littérature (US 3931143, US 4477683 et Wheler O.H. ; De Rodriguez E.G. ; J. Org. Chem., 1964, 29(3), 718-721) et aucune de ces descriptions ne fait état de leur propriétés olfactives.

D'une manière générale, l'industrie de la parfumerie a un besoin constant de nouvelles molécules odorantes afin d'accroître la gamme de notes offerte aux parfumeurs pour leurs créations. De plus, ce besoin est d'autant plus important que l'industrie de la parfumerie a dû faire face à un durcissement de la réglementation internationale ainsi qu'une prise de conscience environnementale et une exigence accrue des consommateurs. L'objet de l'invention est donc de proposer de nouvelles fragrances et/ou composés odorants.

La présente invention concerne de nouveaux aldéhydes et nitriles issus de l'isophorone. Ces composés possèdent l'avantage d'être accessibles par une préparation fiable et peu coûteuse.

La présente invention a donc pour objet les composés de formule générale (I) ci-dessous : 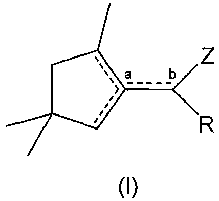 dans laquelle :
R est un atome d'hydrogène, un groupe alkyle en C₁-C₆, un groupe alcényle en C₂-C₆ ou un groupe =CH₂ ;
Z est un groupe CN ou CHO ; et
au plus une des liaisons en pointillé est présente ;
à condition que :
si Z est un groupe CHO et une des liaisons en pointillé est présente, alors R n'est pas un atome d'hydrogène, et
si la liaison en pointillé entre les carbones Ca et Cb est présente, alors R n'est pas un groupe =CH₂.

Par le terme « alkyle en C₁-C₆ » au sens de la présente invention, on entend tout radical monovalent dérivé d'une chaîne carbonée saturée, linéaire ou branchée, contenant 1 à 6 atomes de carbones, notamment les groupes méthyle, éthyle, *n*-propyle, *i*-propyle, *n*-butyle, *i*-butyle, *t*-butyle, pentyle et hexyle. Des groupes alkyles préférés sont les groupes méthyle, éthyle, *n*-propyle, *i*-propyle, *n*-butyle, *i*-butyle et *n*-pentyle.

Par le terme « alcényle en C₂-C₆ » au sens de la présente invention, on entend tout radical monovalent dérivé d'une chaîne carbonée, linéaire ou branchée, contenant 2 à 6 atomes de carbones, comportant au moins une liaison double, notamment les groupes éthényle, *n*-propényle, *i*-propényle, *n*-butényle, *i*-butényle, *t*-butényle, *n*-pentényle et *n-*hexényle. Des groupes alcényles préférés sont les groupes éthényle, *n*-propényle, *i-*propényle, *n*-butényle, *i*-butényle et *n*-pentényle.

La présente invention a également pour objet les stéréoisomères de formule (I), notamment les diastéréoisomères de formule (I), ainsi que les mélanges, en toute proportion, de ces isomères.

En particulier, la présente invention concerne les composés de formule générale (Ia) : 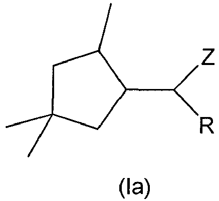 dans laquelle :
R est un atome d'hydrogène, un groupe alkyle en C₁-C₆, un groupe alcényle en C₂-C₆ ou un groupe =CH₂ ;
Z est un groupe CN ou CHO ;
les composés de formule générale (Ib) et (Ic) : 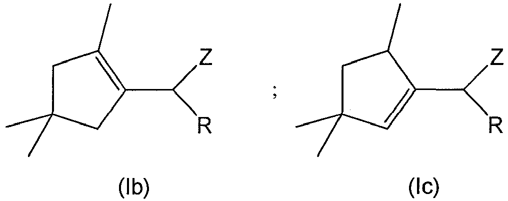 dans lesquelles :
R est un atome d'hydrogène, un groupe alkyle en C₁-C₆, un groupe alcényle en C₂-C₆ ou un groupe =CH₂ ;
Z est un groupe CN ou CHO ; et
à condition que si Z est un groupe CHO alors R n'est pas un atome d'hydrogène ; ceux de formule (Ib) et (Ic), ainsi que les composés de formule générale (Id) : 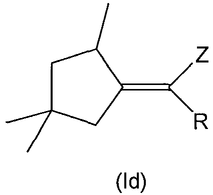
dans laquelle :
R est un atome d'hydrogène, un groupe alkyle en C₁-C₆ ou un groupe alcényle en C₂-C₆ ;
Z est un groupe CN ou CHO ; et
à condition que si Z est un groupe CHO alors R n'est pas un atome d'hydrogène.

Dans un premier mode de réalisation, la présente invention concerne les composés de formule générale (I') : 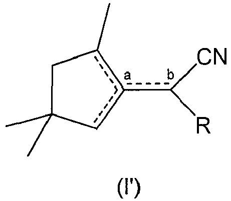 dans laquelle :
R est un atome d'hydrogène, un groupe alkyle en C₁-C₆, alcényle en C₂-C₆ ou un groupe =CH₂ ; de préférence, R est un atome d'hydrogène, un groupe alkyle en C₁-C₆ ou un groupe =CH₂ ; plus préférentiellement, R est un atome d'hydrogène, un groupe méthyle, éthyle, *n*-propyle, *i*-propyle ou =CH₂ ; encore plus préférentiellement R est un atome d'hydrogène, un groupe méthyle ou =CH₂ ; et
au plus une des liaisons en pointillé est présente ;
à condition que si la liaison en pointillé entre les carbones Ca et Cb est présente, alors R n'est pas un groupe =CH₂.

Des composés de formule (I') particulièrement intéressant sont les composés (1) à (6) représentés dans le tableau 1.

Une première variante de ce mode de réalisation concerne les composés de formule (Ia') : 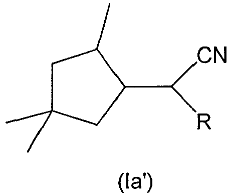 dans laquelle :
R est un atome d'hydrogène, un groupe alkyle en C₁-C₆, alcényle en C₂-C₆ ou un groupe =CH₂ ; de préférence, R est un atome d'hydrogène, un groupe alkyle en C₁-C₆ ou un groupe =CH₂ ; plus préférentiellement, R est un atome d'hydrogène, un groupe méthyle, éthyle, *n*-propyle, *i*-propyle ou =CH₂ ; encore plus préférentiellement R est un atome d'hydrogène, un groupe méthyle ou =CH₂.

Une deuxième variante concerne les composés de formule (Ib') : 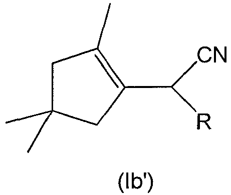 dans laquelle :
R est un atome d'hydrogène, un groupe alkyle en C₁-C₆, alcényle en C₂-C₆ ou un groupe =CH₂ ; de préférence, R est un atome d'hydrogène, un groupe alkyle en C₁-C₆ ou un groupe =CH₂ ; plus préférentiellement, R est un atome d'hydrogène, un groupe méthyle, éthyle, *n*-propyle, *i*-propyle ou =CH₂ ; encore plus préférentiellement R est un atome d'hydrogène, un groupe méthyle ou =CH₂.

Une troisième variante de ce mode de réalisation concerne également les composés de formule (Ic') : 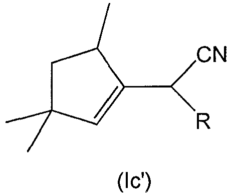 dans laquelle:
R est un atome d'hydrogène, un groupe alkyle en C₁-C₆, alcényle en C₂-C₆ ou un groupe =CH₂ ; de préférence, R est un atome d'hydrogène, un groupe alkyle en C₁-C₆ ou un groupe =CH₂ ; plus préférentiellement, R est un atome d'hydrogène, un groupe méthyle, éthyle, *n*-propyle, *i*-propyle ou =CH₂ ; encore plus préférentiellement R est un atome d'hydrogène, un groupe méthyle ou =CH₂.

Enfin, une dernière variante de ce mode de réalisation concerne les composés de formule (Id') : 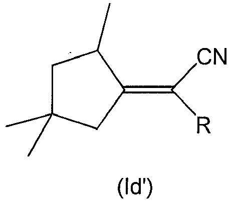 dans laquelle :
R est un atome d'hydrogène, un groupe alkyle en C₁-C₆ ou un groupe alcényle en C₂-C₆ ; de préférence, R est un atome d'hydrogène ou un groupe alkyle en C₁-C₆ ; plus préférentiellement, R est un atome d'hydrogène, un groupe méthyle, éthyle, *n-*propyle ou *i*-propyle ; encore plus préférentiellement R est un atome d'hydrogène ou un groupe méthyle.

Dans un second mode de réalisation, la présente invention concerne également les composés de formule générale (I") : 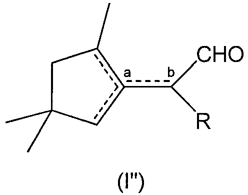 dans laquelle :
R est un atome d'hydrogène, un groupe alkyle en C₁-C₆, alcényle en C₂-C₆ ou un groupe =CH₂ ; de préférence, R est un atome d'hydrogène, un groupe alkyle en C₁-C₆ ou un groupe =CH₂ ; plus préférentiellement, R est un atome d'hydrogène, un groupe méthyle, éthyle, *n*-propyle, *i*-propyle ou =CH₂ ; encore plus préférentiellement R est un atome d'hydrogène, un groupe méthyle ou =CH₂ ; et
au plus une des liaisons en pointillé est présente ;
à condition que si une des liaisons en pointillé est présente, alors R n'est pas un atome d'hydrogène, et
si la liaison en pointillé entre les carbones Ca et Cb est présente, alors R n'est pas un groupe =CH₂.

Des composés de formule (I") particulièrement intéressants sont les composés (7) à (9) représentés dans le tableau 1.

Une première variante de ce mode de réalisation concerne les composés de formule (Ia") : 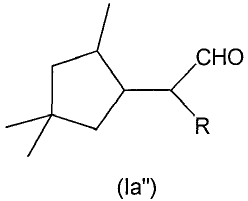 dans laquelle :
R est un atome d'hydrogène, un groupe alkyle en C₁-C₆, alcényle en C₂-C₆ ou un groupe =CH₂ ; de préférence, R est un atome d'hydrogène, un groupe alkyle en C₁-C₆ ou un groupe =CH₂ ; plus préférentiellement, R est un atome d'hydrogène, un groupe méthyle, éthyle, *n*-propyle, *i*-propyle ou =CH₂ ; encore plus préférentiellement R est un atome d'hydrogène, un groupe méthyle ou =CH₂.

Une deuxième variante concerne les composés de formule (Ib") : 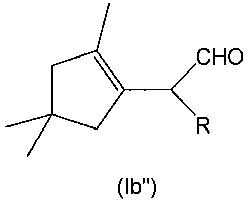 dans laquelle :
R est un groupe alkyle en C₁-C₆, alcényle en C₂-C₆ ou un groupe =CH₂ ; de préférence, R est un groupe alkyle en C₁-C₆ ou un groupe =CH₂ ; plus préférentiellement, R est un groupe méthyle, éthyle, *n*-propyle, *i*-propyle ou =CH₂ ;
encore plus préférentiellement R est un groupe méthyle ou =CH₂.

Une troisième variante de ce mode de réalisation concerne également les composés de formule (Ic") : 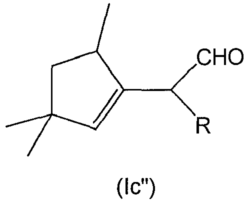 dans laquelle :
R est un groupe alkyle en C₁-C₆, alcényle en C₂-C₆ ou un groupe =CH₂ ; de préférence, R est un groupe alkyle en C₁-C₆ ou un groupe =CH₂ ; plus préférentiellement, R est un groupe méthyle, éthyle, *n*-propyle, *i*-propyle ou =CH₂ ;
encore plus préférentiellement R est un groupe méthyle ou =CH₂.

Enfin, une dernière variante de ce mode de réalisation concerne les composés de formule (Id") : 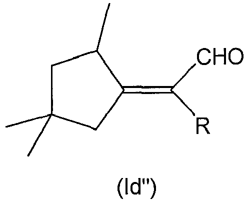 dans laquelle :
R est un groupe alkyle en C₁-C₆ ou alcényle en C₂-C₆ ; de préférence, R est un groupe alkyle en C₁-C₆ ; plus préférentiellement, R est un groupe méthyle, éthyle, *n*-propyle ou *i*-propyle ; encore plus préférentiellement R est un groupe méthyle.

Plus particulièrement, l'invention concerne les composés de formule (I) représentés dans le tableau 1.

**Tableau 1**

| composé | formule | composé | Formule |
|---|---|---|---|
| 1 | 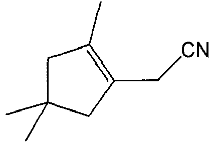 | 2 | 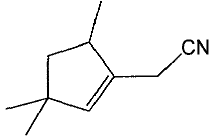 |
| 3 | 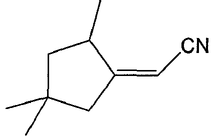 | 4 | 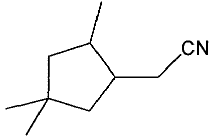 |
| 5 | 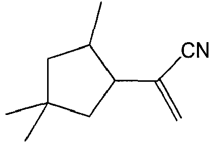 | 6 | 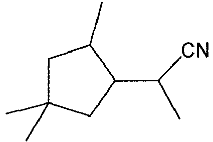 |
| 7 | 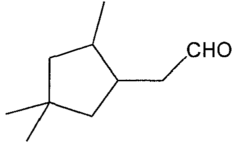 | 8 | 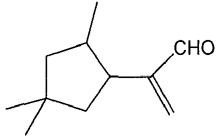 |
| 9 | 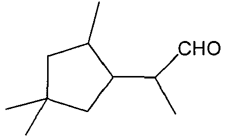 | | |

L'invention comprend tous les énantiomères et diastéréoisomères des composés de formule (I), seuls ou en mélange. La présence dans la structure des composés de formule (I) de centres d'asymétrie provoque l'existence, pour chacun d'entre eux, de formes énantiomériques. L'invention comprend les composés représentés par la formule générale (I) sous forme de mélanges des énantiomères en proportions variables, en particulier les mélanges racémiques. L'invention comprend également les composés de formule (I) sous forme d'un seul énantiomère. L'élaboration des mélanges d'énantiomères ou de formes pures est réalisée par des méthodes connues de l'homme de l'art en utilisant par exemple des produits de départ optiquement enrichis ou optiquement purs.

Les composés de formule (I) possèdent l'avantage d'être accessibles par une préparation fiable et peu coûteuse. Ainsi, les composés de formule (I) peuvent être obtenus à partir de l'isophorone.

La présente invention concerne également un procédé de préparation d'un composé de formule (I) comprenant une étape consistant à faire réagir le composé de formule (A) 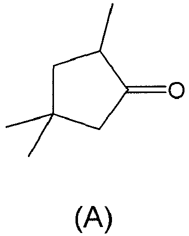 avec un diéthylphosphonodialkylacétonitrile de formule RCH(CN)PO(OC₂H₅)₂ dans laquelle R est un atome d'hydrogène, un groupe alkyle en C₁-C₆ ou un groupe alcényle en C₂-C₆ ; de préférence, R est un atome d'hydrogène ou un groupe alkyle en C₁-C₆ ; plus préférentiellement, R est un atome d'hydrogène, un groupe méthyle, éthyle, *n*-propyle ou *i*-propyle ; encore plus préférentiellement R est un atome d'hydrogène ou un groupe méthyle ; ou

avec un cyanoacétate d'alkyl de formule NCCH₂COOR' dans laquelle R' est un groupe alkyl en C₁-C₄ ; de préférence, R' est un groupe méthyle.

Dans un premier temps, l'époxyde d'isophorone est obtenu à partir de l'isophorone par une époxydation de Weitz-Scheffer. Le réarrangement de l'époxyde d'isophorone en milieu acide conduit à la 2,4,4-triméthylcyclopentanone (A).

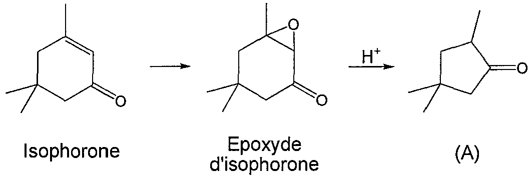

Dans un premier mode de réalisation, les composés de formule (Ia') et (Id') sont obtenus selon le schéma 1 :

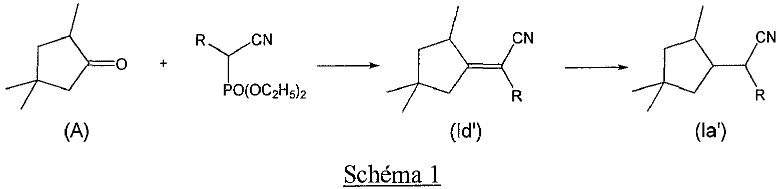

L'action d'un diéthylphosphonoalkylacétonitrile de formule RCH(CN)PO(OC₂H₅)₂, R étant tel que défini plus haut, sur la cyclopentanone de formule (A) conduit aux composés de formule (Id'). Cette réaction est avantageusement réalisée en présence d'une base telle qu'un organolithié, de préférence le butyllithium (BuLi), dans un solvant apolaire comme le tétrahydrofuranne. L'hydrogénation des composés de formule (Id') permet d'obtenir les composés de formules (Ia') dans laquelle R ne peut pas être =CH₂. L'hydrogénation est réalisée dans les conditions bien connues de l'homme du métier, par exemple dans un autoclave sous atmosphère d'hydrogène en présence d'un catalyseur de palladium sur charbon à 5%.

Dans un second mode de réalisation, les composés de formule (Ib') et (Ic') ainsi que les composés (5) et (6), sont obtenus selon le schéma 2 :

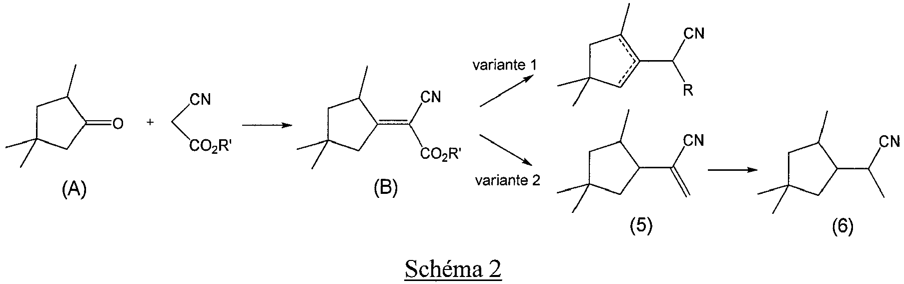

Dans un premier temps, la cyclopentanone de formule (A) est condensée avec un cyanoacétate d'alkyle de formule NCCH₂COOR', dans laquelle R' est un groupe alkyle en C₁-C₄, de préférence un groupe méthyle, pour donner le composé de formule (B). La condensation est par exemple réalisée en présence d'acide acétique (AcOH) et d'acétate d'ammonium (AcONH₄) dans un solvant apolaire aprotique tel que le cyclohexane à température de reflux.

Dans une première variante, le composé (B) subit ensuite une décarboxylation éventuellement suivie d'une hydrogénation et/ou d'une alkylation pour conduire aux composés de formule (Ia'), (Ib') ou (Ic'). La décarboxylation est avantageusement réalisée en présence d'un halogénure alcalin tel que le chlorure de lithium dans un solvant polaire comme la N-méthylpyrrolidone. L'hydrogénation est réalisée dans les conditions bien connues de l'homme du métier, par exemple dans un autoclave sous atmosphère d'hydrogène en présence d'un catalyseur de palladium sur charbon à 5%. L'alkylation peut être réalisée à l'aide d'un agent d'alkylation choisi parmi un bromure d'alkyle, un iodure d'alkyle ou un chlorure d'alkyle et d'une base comme par exemple le diisopropylamidure de lithium ou le *tert*-butylate de potassium. Un solvant adéquat pour cette réaction peut être un solvant apolaire comme le tétrahydrofuranne ou un solvant polaire aprotique tel que le diméthylformamide.

Dans une seconde variante, la réduction du composé de formule (B) suivie d'une déshydratation et éventuellement d'une hydrogénation permet d'obtenir les composés (5) et (6). La réduction est avantageusement réalisée grâce à un hydrure tel que NaBH₄. L'hydrogénation est réalisée dans les conditions bien connues de l'homme du métier, par exemple dans un autoclave sous atmosphère d'hydrogène en présence d'un catalyseur de palladium sur charbon à 5%.

D'une manière générale, les composés de formule (I") sont obtenus par la réduction ménagée de la fonction nitrile des composés de formule (I') correspondants en fonction aldéhyde. Cette réduction peut être réalisée par exemple en présence d'hydrure de diisobutylaluminium (Dibal) dans un solvant apolaire tel que le toluène.

De par leurs propriétés odorantes, les composés de formule (I) trouvent un emploi très varié en parfumerie, notamment et à titre non limitatif en cosmétique et pour les produits d'entretien.

L'invention a donc également pour objet l'utilisation d'au moins un composé de formule (I) selon l'invention comme agent ou composé fragrant, comme agent de masquage d'odeur ou comme agent de neutralisation d'odeur, seul ou en mélange avec un ou plusieurs autres composés odorants connus de l'homme du métier, et que l'homme du métier est à même de choisir en fonction de l'effet recherché. Le ou les agents odorants supplémentaires peuvent être des composés de formule (I) ou d'autres agents odorants connus de l'homme du métier.

Pour les mêmes raisons, l'invention porte également sur une méthode pour parfumer un produit de base comprenant l'ajout d'un composé de l'invention au dit produit de base. Le composé de l'invention peut être ajouté seul ou en mélange avec un ou plusieurs autres composés odorants connus de l'homme du métier, et que l'homme du métier est à même de choisir en fonction de l'effet recherché. Le ou les agents odorants supplémentaires peuvent être des composés de formule (I) ou d'autres agents odorants connus de l'homme du métier. Ledit produit de base peut être en particulier une composition de parfumerie, notamment une base ou un concentré parfumant, une eau de Cologne, une eau de toilette ou un parfum ; une composition cosmétique, notamment crème pour le visage et le corps, poudre de talc, huile pour cheveux ou pour le corps, shampoing, lotion capillaire, sel de bain, huile de bain, gel de douche, gel de bain, savon de toilette, antitranspirant corporel, désodorisant corporel, lotions, crème de rasage, savon de rasage, crème, dentifrice, bain de bouche ou pommade ; ou un produit d'entretien, notamment assouplissant, détergent, lessive ou désodorisant d'ambiance.

L'invention a également pour objet les compositions comprenant un produit de base et une quantité efficace d'un ou plusieurs composés de formule (I) selon l'invention.

Il peut s'agir d'une composition elle-même odorante, ou d'une composition dans laquelle l'agent odorant est utilisé pour masquer ou neutraliser certaines odeurs.

Le produit de base sera aisément déterminé par l'homme du métier en fonction de la composition envisagée et donc de l'utilisation envisagée, pour lesquelles les composants habituels, tels que solvant(s) et/ou adjuvant(s), sont bien connus.

La quantité efficace des composés de formule (I) selon l'invention incorporée dans la composition variera selon la nature de la composition, l'effet odorant souhaité, et la nature des autres composés odorants ou non éventuellement présents, et pourra être aisément déterminée par l'homme du métier, sachant qu'elle peut varier dans une plage très étendue, de 0,1 à 99% en poids, en particulier 0,1 à 50% en poids, notamment 0,1 à 30% en poids.

Les composés de formule (I) selon l'invention peuvent être utilisés tels quels ou ils peuvent être incorporés dans ou sur un matériau support inerte ou qui peut contenir d'autres ingrédients actifs de la composition finie. Une grande variété de matériaux supports peut être employée, incluant, par exemple, les solvants polaires, les huiles, les graisses, les solides finement divisés, les cyclodextrines, les maltodextrines, les gommes, les résines et n'importe quel autre matériau support connu pour de telles compositions.

L'invention a donc aussi pour objet l'utilisation des composés de formule (I) pour la préparation d'une composition odorante ou d'un article odorant dans les applications décrites ci-dessus, en particulier en parfumerie, en cosmétique, par exemple pour des shampooings ou des savons, et pour des produits d'entretien, tels que des assouplissants ou des lessives.

L'invention concerne en particulier une composition de parfumerie, notamment une base ou un concentré parfumant, une eau de Cologne, une eau de toilette ou un parfum, comprenant au moins un composé de formule (I) ou une composition comprenant au moins un composé de formule (I).

L'invention concerne également en particulier une composition cosmétique, notamment crème pour le visage et le corps, poudre de talc, huile pour cheveux ou pour le corps, shampoing, lotion capillaire, sel de bain, huile de bain, gel de douche, gel de bain, savon de toilette, antitranspirant corporel, désodorisant corporel, lotions, crème de rasage, savon de rasage, crème, dentifrice, bain de bouche, pommade comprenant au moins un composé de formule (I) ou au moins une composition comprenant au moins un composé de formule (I). Un autre objet de l'invention est une méthode de traitement ou de soin cosmétique, préventive ou non, mettant en oeuvre au moins un composé de formule (I) ou au moins une composition comprenant au moins un composé de formule (I).

L'invention concerne encore un produit d'entretien, notamment assouplissant, détergent, lessive, désodorisant d'ambiance, comprenant au moins un composé de formule (I) ou au moins une composition comprenant au moins un composé de formule (I).

Les composés selon l'invention peuvent être utilisé, seuls ou en combinaison, tels quels ou être incorporés dans ou sur un matériau support inerte ou qui peut contenir d'autres ingrédients actifs de la composition finie. Une grande variété de matériaux supports peut être employée incluant, par exemple, les solvants polaires, les huiles, les graisses, les solides finement divisés, les cyclodextrines, les maltodextrines, les gommes, les résines et n'importe quel autre matériau support connu pour de telles compositions.

Les exemples suivants illustrent davantage les différents procédés de fabrication des composés nouveaux selon l'invention ainsi que leur utilisation, et leur intérêt. Ces exemples ne sont présentés que dans un but d'illustration et ne peuvent être considérés comme limitatifs de l'invention.

### Exemple 1 : Préparation de 2-(2,4,4-triméthylcyclopent-1-ènyl)acétonitrile (1) et 2-(3,3,5-triméthylcyclopent-1-ènyl)acétonitrile (2)

On place dans le ballon la cyclopentanone **A,** 1,5 éq. de cyanoacétate de méthyle, 1 éq. d'acide acétique et 10 % molaire d'acétate d'ammonium, dans le cyclohexane. Le milieu réactionnel est porté à reflux afin d'éliminer l'eau formée à l'aide d'un appareillage de type Dean-Stark. Après une nuit à reflux, la conversion de **A** est satisfaisante (>90%), on laisse donc le milieu réactionnel revenir à température ambiante. On lave avec une solution de bicarbonate de sodium puis à l'eau salée. Après séchage sur sulfate de magnésium, filtration sur papier et évaporation du solvant, le produit brut **B** est distillé sous pression réduite.

Téb. = 80 °C / 0,3 torr

Le 2-cyano-2-(2,4,4-triméthylcyclopentylidène)acétate de méthyle **B** est placé dans un ballon avec 2 éq. de chlorure de lithium dans un mélange 98 : 2 de NMP (N-méthylpyrrolidone) et d'eau. On agite à 150 °C. Après 3 heures, on refroidit le milieu réactionnel à température ambiante avant de le verser sur une solution de HCl à 1%. On agite quelques minutes puis on extrait deux fois au MTBE (éther de *tert*-butyle et de méthyle). Les phases organiques réunies sont lavées avec une solution de bicarbonate de sodium puis à l'eau salée. Après séchage sur sulfate de magnésium, filtration sur papier et évaporation du solvant, le produit brut est distillé sous pression réduite. On obtient un mélange 70 : 30 de 2-(2,4,4-triméthylcyclopent-1-ènyl)acétonitrile et de 2-(3,3,5-triméthylcyclopent-1-ènyl)acétonitrile.

Téb. = 78-82 °C / 6 torr

Description olfactive : épicé, cumin, boisé, citrus, puissant.

### Isomère majoritaire :

**¹H-NMR** (200 MHz, CDCl₃) : δ (ppm) 1,08 (s, 6H), 1,63 (s, 3H), 2,15 (s, 2H), 2,25 (s, 2H), 3,08 (s, 2H).
**¹³C**-**NMR** (50 MHz, CDCl₃) : δ (ppm) 17,43, 19,82, 30,07, 30,19, 50,99, 53,84, 121,95, 136,53, 136,54.
**MS** [e/m (%)] : 149 (M+, 45), 135 (10), 134 (100), 109 (43), 108 (15), 107 (27), 94 (21), 93 (66), 91 (26), 79 (22), 77 (18), 67 (16), 41 (14), 39 (13).

### Isomère minoritaire :

¹**H**-**NMR** (200 MHz, CDCl₃) : δ (ppm) 1,02-1,04 (m, 3H), 1,08 (s, 6H), 1,10-1,20 (m, 2H), 1,95-2,05 (m, 1H), 3,01 (s, 2H), 5,56 (s, 1H).
¹³**C**-**NMR** (50 MHz, CDCl₃) : δ (ppm) 14,40, 18,22, 28,52, 36,70, 40,77, 48,92, 118,08, 134,15, 139,95.
**MS** [e/m (%)] : 149 (M+, 53), 148 (22), 135 (11), 134 (94), 122 (10), 120 (12), 109 (26), 108 (35), 107 (48), 106 (22), 94 (38), 93 (48), 92 (15), 91 (27), 83 (100), 80 (19), 79 (27), 77 (23), 69 (37), 67 (21), 66 (30), 65 (22), 56 (20), 55 (33), 54 (10), 53 (15), 51 (11), 43 (10), 41 (41), 39 (31).

IR (film, cm⁻¹) : 759w, 840w, 931w, 1070 w, 1159w, 1241w, 1315w, 1364m, 1415m, 1446m, 2249m, 2837m, 2866m, 2954s.

### Exemple 2 : Préparation de 2-(2,4,4-triméthylcyclopentylidène)acétonitrile (3)

On place sous atmosphère inerte 1,1 éq. de diéthylphosphonoacétonitrile dans le THF (tétrahydrofuranne). On y ajoute goutte à goutte vers 10 °C 1,1 éq. d'une solution du butyllithium dans l'hexane. On agite ensuite une heure à température ambiante avant d'ajouter goutte à goutte vers 10 °C 1 éq. de cyclopentanone **A**. Le milieu réactionnel est agité quelques heures à température ambiante, puis versé sur une solution de HCl à 10%. On extrait trois fois la phase aqueuse au MTBE. On lave les phases organiques réunies avec une solution de bicarbonate de sodium puis à l'eau salée. Après séchage sur sulfate de magnésium, filtration sur papier et évaporation du solvant, le produit brut est distillé sous pression réduite. On obtient un mélange 75 : 25 des isomères *trans* et *cis* du 2-(2,4,4-triméthylcyclopentylidène)acétonitrile.

Téb.=85 °C/6torr

Description olfactive : frais, citrus, agrume, vert, puissant.

### Isomère majoritaire :

¹**H**-**NMR** (200 MHz, CDCl₃) : δ (ppm) 0,99 (s, 3H), 1,12 (s, 6H), 1,20-1,32 (m, 2H), 1,72-1,95 (m, 1H), 2,23-2,61 (m, 1H), 2,76-2,82 (m, 1H), 5,12 (s, 1H).
**¹³C**-**NMR** (50 MHz, CDCl₃) : δ (ppm) 18,88, 28,01, 29,37, 37,84, 39,22, 48,59, 49,41, 91,17, 117,93, 178,84.
**MS** [e/m (%)] : 149 (M+, 47), 148 (21), 135 (11), 134 (90), 122 (11), 120 (12), 109 (22), 108 (34), 107 (42), 106 (18), 95 (10), 94 (35), 93 (49), 92 (15), 91 (22), 84 (10), 83 (100), 81 (15), 80 (19), 79 (26), 78 (10), 77 (20), 69 (33), 67 (19), 66 (29), 65 (21), 56 (22), 55 (35), 53 (15), 51 (11), 43 (10), 41 (40), 39 (30).

### Isomère minoritaire :

¹**H**-**NMR** (200 MHz, CDCl₃) : δ (ppm) 0,89 (s, 3H), 1,14 (s, 6H), 1,20-1,32 (m, 2H), 1,72-1,95 (m, 1H), 2,23-2,61 (m, 1H), 2,76-2,82 (m, 1H), 5,24 (s, 1H).
**¹³C**-**NMR** (50 MHz, CDCl₃) : δ (ppm) 19,93, 27,24, 28,88, 38,06, 38,58, 49,63, 50,18, 91,50, 117,93, 178,84.
**MS** [e/m (%)] : 149 (M+, 12), 135 (10), 134 (100), 109 (17), 107 (11), 94 (20), 93 (82), 91 (21), 79 (20), 77 (14), 41 (12).
**IR** (film, cm⁻¹) : 770w, 795m, 809s, 880w, 996w, 1268w, 1320w, 1369m, 1387m, 1420m, 1463s, 1637s, 2216s, 2870m, 2956s.

### Exemple 3 : Préparation du 2-(2,4,4-triméthylcyclopentyl)acétonitrile (4)

Une solution contenant 1 équivalent d'un mélange des composés **1** et **2** obtenu à l'exemple 1 dans le toluène et 5% en masse de palladium sur charbon à 5% est placée dans un autoclave, sous 20 bars d'hydrogène, à 40 °C. A la fin de la réaction, l'autoclave est purgé à l'azote et la solution filtrée sur célite. Le filtrat est concentré. Le produit brut obtenu sous forme de deux diastéréomères en proportions 70 : 30 est purifié par distillation.

Téb. = 80 °C / 6 torr

Description olfactive : vert, frais, zest, puissant.

### Isomère majoritaire :

**¹H-NMR** (200 MHz, CDCl₃) : δ (ppm) 0,92-1,08 (m, 3H), 1,04 (s, 6H), 1,15-1,42 (m, 2H), 1,60-1,87 (m, 3H), 2,23-2,44 (m, 3H).
**¹³C-NMR** (50 MHz, CDCl₃) : δ (ppm) 18,82, 21,33, 31,54, 37,09, 39,94, 43,66, 47,86, 50,59, 119,42.
**MS** [e/m (%)] : 151 (M+, 0,3), 136 (39), 119 (20), 111 (21), 108 (16), 96 (13), 95 (100), 83 (67), 82 (26), 69 (30), 67 (18), 56 (11), 55 (25), 41 (28), 39 (14).

### Isomère minoritaire :

**¹H-NMR** (200 MHz, CDCl₃) : δ (ppm) 0,92-1,08 (m, 3H), 1,04 (s, 6H), 1,15-1,42 (m, 2H), 1,60-1,87 (m, 3H), 2,23-2,44 (m, 3H).
**¹³C-NMR** (50 MHz, CDCl₃) : δ (ppm) 16,16, 19,29, 30,43, 35,47, 37,09, 39,04, 46,71, 49,42, 120,36.
**MS** [e/m (%)] : 151 (M+, 0,2), 136 (25), 119 (13), 111 (29), 109 (10), 108 (20), 96 (27), 95 (100), 94 (11), 84 (16), 83 (69), 82 (31), 81 (11), 69 (52), 68 (10), 67 (20), 56 (24), 55 (29), 53 (11), 41 (34), 39 (17).
**IR** (film, cm⁻¹) : 931w, 1064w, 1316w, 1366m, 1379m, 14124, 1463m, 2246m, 2866m, 2930s, 2952s.

### Exemple 4 : Préparation du 2-(2,4,4-triméthylcyclopentyl)acrylonitrile (5)

1,1 éq. de borohydrure de sodium est placé sous atmosphère inerte dans l'éthanol, à 10 °C. On y ajoute goutte à goutte le 2-cyano-2-(2,4,4-triméthylcyclopentylidène)acétate de méthyle **B**. Le milieu réactionnel est agité une nuit à température ambiante, puis neutralisé avec de l'acétone. Il est ensuite versé sur une solution froide de HCl 10%. On extrait deux fois la phase aqueuse au MTBE. On lave les phases organiques réunies avec une solution de bicarbonate de sodium puis à l'eau salée. Après séchage sur sulfate de magnésium, filtration sur papier et évaporation du solvant, du 3-hydroxy-2-(2,4,4-triméthylcyclopentyl)propanenitrile est obtenu sous forme de quatre diastéréomères en proportions 11 : 13 : 23 : 53.

Le 3-hydroxy-2-(2,4,4-triméthylcyclopentyl)propanenitrile est placé sous atmosphère inerte dans le dichlorométhane, à 10 °C. On y ajoute 2,2 éq. de DBU (diazabicyclo[5.4.0]undec-7-ène), et quelques cristaux de DMAP (4-diméthylaminopyridine), puis goutte à goutte 1,2 éq. d'anhydride trifluoroacétique. Le milieu réactionnel est agité une nuit à température ambiante, puis versé sur de l'eau. On extrait une fois la phase aqueuse au dichlorométhane. On lave les phases organiques réunies avec de l'eau. Après séchage sur sulfate de magnésium, filtration sur papier et évaporation du solvant, le produit brut obtenu sous forme de deux diastéréomères en proportions 70 : 30 est distillé sous pression réduite.

Téb. = 35 °C / 0,3 torr

Description olfactive : vert, fruité, peau de pêche, miel.

### Isomère majoritaire :

**¹H-NMR** (200 MHz, CDCl₃) : δ (ppm) 0,85 (d, *J* = 7,2 Hz, 3H), 0,96-1,06 (m, 6H), 1,10-1,17 (m, 2H), 1,64-1,83 (m, 4H), 2,17-2,48 (m, 1H), 2,55-2,64 (m, 1H).
**¹³C-NMR** (50 MHz, CDCl₃) : δ (ppm) 18,94, 31,70, 40,84, 42,07, 46,98, 48,61, 49,20, 50,39, 203,41.
**MS** [e/m (%)] : 163 (M+, 1), 148 (21), 121 (10), 120 (12), 107 (10), 106 (40), 84 (100), 83 (15), 79 (21), 77 (13), 55 (16), 41 (21), 39 (13).

### Isomère minoritaire :

**¹H-NMR** (200 MHz, CDCl₃) : δ (ppm) 0,85 (d, *J* = 7,2 Hz, 3H), 0,96-1,06 (m, 6H), 1,10-1,17 (m, 2H), 1,64-1,83 (m, 4H), 2,17-2,48 (m, 1H), 2,55-2,64 (m, 1H).
**¹³C-NMR** (50 MHz, CDCl₃) : δ (ppm) 16,94, 30,64, 35,64, 36,46, 46,12, 48,51, 49,67, 50,65, 203,41.
**MS** [e/m (%)] : 163 (M+, 0,4), 148 (12), 106 (29), 84 (100), 79 (17), 77 (11), 69 (46), 55 (11), 41 (18), 39 (11).
**IR** (film, cm⁻¹) : 841w, 932s, 1078w, 1195w, 1294w, 1367m, 1378m, 1407w, 1461m, 1618m, 2222m, 2867m, 2953s.

### Exemple 5 : Préparation du 2-(2,4,4-triméthylcyclopentyl)propanenitrile (6)

Une solution contenant 1 équivalent de 2-(2,4,4-triméthylcyclopentyl)acrylonitrile **5** dans le toluène et 5% en masse de palladium sur charbon à 5% est placée dans un autoclave, sous 20 bars d'hydrogène, à 40 °C. A la fin de la réaction, l'autoclave est purgé à l'azote et la solution filtrée sur célite. Le filtrat est concentré. Le produit brut obtenu sous forme de quatre diastéréomères en proportions 8 : 18 : 22 : 52 est purifié par distillation.

Téb. = 46 °C / 0,7 torr

Description olfactive : vert, agreste, armoise, boisé.

### 4 Isomères superposés :

**¹H-NMR** (200 MHz, CDCl₃) : δ (ppm) 0,91-1,03 (m, 6H), 1,09-1,11 (m, 3H), 1,31-1,92 (m, 2H), 1,25-1,39 (m, 3H), 1,72-1,74 (m, 1H), 2,21-2,43 (m, 1H), 2,59-2,84 (m, 1H).
**¹³C-NMR** (50 MHz, CDCl₃, isomère majoritaire (52%)) : δ (ppm) 16,51, 20,91, 28,73, 30,88, 30,99, 35,95, 38,42, 46,23, 49,64, 50,78, 123,32.
**MS** [e/m (%)], isomère à 52% : 165 (M+, 1), 150 (49), 133 (32), 111 (65), 109 (10), 96 (19), 95 (100), 83 (54), 81 (14), 69 (69), 67 (14), 56 (11), 55 (43), 53 (11), 41 (32), 39 (14).
**MS** [e/m (%)], isomère à 22% : 165 (M+, 0,4), 150 (57), 133 (26), 111 (33), 96 (14), 95 (100), 83 (68), 81 (11), 69 (47), 67 (12), 55 (34), 41 (25), 39 (12).
**MS** [e/m (%)], isomère à 18% : 165 (M+, 0,3), 150 (31), 133 (17), 122 (11), 111 (84), 110 (17), 109 (19), 96 (19), 95 (83), 94 (11), 84 (29), 83 (54), 82 (13), 81 (15), 69 (100), 67 (17), 56 (18), 55 (49), 53 (13), 41 (40), 39 (17).
**MS** [e/m (%)], isomère à 8% : 165 (M+, 0,1), 150 (29), 133 (19), 111 (87), 110 (16), 109 (18), 96 (15), 95 (83), 94 (14), 84 (32), 83 (56), 82 (13), 81 (17), 69 (100), 67 (15), 56 (15), 55 (49), 53 (11), 41 (38), 39 (16).
**IR** (film, cm⁻¹) : 979w, 1103w, 1197w, 1319w, 1366m, 1381m, 1461m, 2238m, 2867m, 2952s.

### Exemple 6 : Préparation du 2-(2,4,4-triméthyleyclopentyl)acétaldéhyde (7)

Le 2-(2,4,4-triméthylcyclopentyl)acétonitrile 4 est placé sous atmosphère inerte dans le toluène, à 10 °C. On y ajoute goutte à goutte 1,2 éq. de Dibal (hydrure de diisobutylaluminium). Le milieu réactionnel est agité quelques heures à température ambiante, puis versé sur un mélange 20 : 40 : 40 d'acide acétique, d'eau et de glace. On extrait une fois la phase aqueuse au toluène. On lave les phases organiques réunies avec une solution de bicarbonate de sodium puis à l'eau salée. Après séchage sur sulfate de magnésium, filtration sur papier et évaporation du solvant, le produit brut obtenu sous forme de deux diastéréomères en proportions 70 : 30 est distillé sous pression réduite.

Téb. = 50 °C / 0,3 torr

Description olfactive : très vert, citrus, épicé, cumin, très puissant.

### Isomère majoritaire :

**¹H-NMR** (200 MHz, CDCl₃) : δ (ppm) 1,05 (2d, J = 7,0 Hz et J = 6,8 Hz, 6H), 1,57-1,64 (m, 2H), 1,94-1,98 (m, 1H), 2,28-2,35 (m, 1H), 2,52-2,71 (m, 2H), 7,14-7,28 (m, 5H), 9,65 (s, 1H).
**¹³C-NMR** (50 MHz, CDCl₃) : δ (ppm) 10,26, 17,71, 33,71, 33,91, 35,43, 51,88, 126,27, 128,71 (2C), 128,81 (2C), 142,49, 205,95.
**MS [e/m (%)] :** 154 (M+, 2), 110 (35), 95 (100), 69 (21), 55 (17), 41 (17).

### Isomère minoritaire :

**¹H-NMR** (200 MHz, CDCl₃) : δ (ppm) 0,89 (2d, *J =* 7,0 Hz et *J* = 6,8 Hz, 6H), 1,57-1,64 (m, 2H), 1,94-1,98 (m, 1H), 2,28-2,35 (m, 1H), 2,52-2,71 (m, 2H), 7,14-7,28 (m, 5H), 9,64 (s, 1H).
**¹³C-NMR** (50 MHz, CDCl₃) : δ (ppm) 8,57, 15,78, 32,59, 34,02, 36,97, 50,93, 126,27, 128,71 (2C), 128,81 (2C), 142,49, 205,95.
**MS** [e/m (%)] :154 (M+, 0,5), 110 (36), 109 (13), 95 (100), 83 (11), 69 (33), 55 (21), 41 (22).
**IR** (film, cm⁻¹) : 698s, 747m, 1454m, 1496m, 1603w, 1721s, 2704w, 2860m, 2933m, 2963m, 3026m, 3062w.

### Exemple 7 : Préparation du 2-(2,4,4-triméthylcyclopentyl)acrylaldéhyde (8)

Le 2-(2,4,4-triméthylcyclopentyl)acrylonitrile 5 est placé sous atmosphère inerte dans le toluène, à 10 °C. On y ajoute goutte à goutte 1,2 éq. de Dibal (hydrure de diisobutylaluminium). Le milieu réactionnel est agité quelques heures à température ambiante, puis versé sur un mélange 20 : 40 : 40 d'acide acétique, d'eau et de glace. On extrait une fois la phase aqueuse au toluène. On lave les phases organiques réunies avec une solution de bicarbonate de sodium puis à l'eau salée. Après séchage sur sulfate de magnésium, filtration sur papier et évaporation du solvant, le produit brut obtenu sous forme de deux diastéréomères en proportions 70 : 30 est distillé sous pression réduite.

Téb. = 40 °C / 0,3 torr

Description olfactive : vert, citrus, boisé, puissant.

### Isomère majoritaire :

**¹H-NMR** (200 MHz, CDCl₃) : δ (ppm) 0,88 (d, *J =* 6,4 Hz, 3H), 1,05 (2s, 6H), 1,22-1,34 (m, 2H), 1,60-1,87 (m, 2H), 2,04-2,17 (m, 1H), 2,57-2,67 (m, 1H), 6,01 et 6,28 (2s, 2H), 9,56 (s, 1H).
**¹³C-NMR** (50 MHz, CDCl) : δ (ppm) 18,61, 31,78, 37,28, 39,56, 45,53, 49,06, 50,60, 133,39, 153,26, 195,50.
**MS** [e/m (%)] : 166 (M+, 35), 151 (89), 137 (30), 133 (43), 124 (17), 123 (60), 110 (26), 109 (100), 108 (21), 107 (21), 105 (20), 96 (12), 95 (88), 93 (25), 91 (24), 84 (29), 83 (39), 82 (11), 81 (58), 79 (32), 77 (22), 70 (12), 69 (57), 68 (11), 67 (39), 65 (11), 56 (10), 55 (42), 53 (27), 43 (16), 41 (58), 39 (31).

### Isomère minoritaire :

**¹H-NMR** (200 MHz, CDCl₃ : δ (ppm) 0,59 (d, *J=* 7,2 Hz, 3H), 1,05 (2s, 6H), 1,22-1,34 (m, 2H), 1,60-1,87 (m, 2H), 2,04-2,17 (m, 1H), 2,57-2,67 (m, 1H), 6,07 et 6,20 (2s, 2H), 9,56 (s, 1H).
**¹³C-NMR** (50 MHz, CDCl) : δ (ppm) 18,13, 31,54, 34,56, 37,28, 40,47, 43,24, 49,74, 134,33, 151,82, 195,50.
**MS** [e/m (%)] : 166 (M+, 25), 151 (66), 137 (27), 133 (29), 124 (14), 123 (44), 110 (20), 109 (100), 108 (18), 107 (17), 105 (14), 95 (68), 93 (20), 91 (22), 84 (72), 83 (39), 82 (10), 81 (57), 79 (29), 70 (11), 69 (86), 67 (32), 65 (10), 56 (12), 55 (39), 53 (23), 43 (13), 41 (57), 39 (29).
**IR** (film, cm⁻¹) : 895w, 937m, 1190w, 1242w, 1364w, 1376w, 1459m, 1623w, 1693s, 2696w, 2865m, 2952m.

### Exemple 8 : Préparation du 2-(2,4,4-triméthylcyclopentyl)propanal (9)

Le 2-(2,4,4-triméthylcyclopentyl)propanenitrile **6** est placé sous atmosphère inerte dans le toluène, à 10 °C. On y ajoute goutte à goutte 1,2 éq. de Dibal (hydrure de diisobutylaluminium). Le milieu réactionnel est agité quelques heures à température ambiante, puis versé sur un mélange 20 : 40 : 40 d'acide acétique, d'eau et de glace. On extrait une fois la phase aqueuse au toluène. On lave les phases organiques réunies avec une solution de bicarbonate de sodium puis à l'eau salée. Après séchage sur sulfate de magnésium, filtration sur papier et évaporation du solvant, le produit brut obtenu sous forme de trois diastéréomères observés en CPG en proportions 73 : 16 : 11 est distillé sous pression réduite.

Téb. = 40 °C / 0,4 torr

Description olfactive : boisé, sec, vert, citrus, frais.

### 3 Isomères superposés :

**¹H-NMR** (200 MHz, CDCl₃) : δ (ppm) 0,95-1,03 (s superposés, 6H), 1,07-1,11 (d superposés, 3H), 1,17-1,32 (m, 2H), 1,40-1,50 (m, 1H), 1,62-1,71 (m, 1H), 1,81-2,05 et 2,20-2,23 (2m, 2H), 2,35-2,48 (m, 1H), 9,64 et 9,70 (2s, 1H).
**¹³C-NMR** (50 MHz, CDCl₃, isomère à 73%) : δ (ppm) 9,43, 19,34, 31,21, 31,40, 37,0, 37,40, 45,90, 47,10, 48,65, 50,46, 205,88.
**MS** [e/m (%), isomère à 73%] : 168 (M+, 1), 111 (14), 110 (49), 95 (100), 83 (26), 69 (30), 58 (10), 55 (27), 41 (17).
**MS** [e/m (%), isomère à 16%] : 168 (M+, 0,1), 111 (19), 110 (71), 97 (13), 95 (100), 83 (40), 69 (52), 67 (10), 58 (11), 55 (42), 43 (11), 41 (24).
**MS** [e/m (%), isomère à 10%] : 168 (M+, 1), 111 (20), 110 (37), 109 (12), 95 (100), 83 (79), 82 (15), 69 (45), 67 (13), 58 (33), 55 (45), 43 (12), 41 (27), 39 (10).
**MS** [e/m (%)] :190 (M+, 18), 132 (11), 131 (39), 117 (33), 105 (16), 104 (33), 92 (25), 91 (100), 85 (31), 65 (14).
**IR** (film, cm⁻¹) : 840w, 885w, 920w, 971w, 1365w, 1377w, 1460m, 1725s, 2700w, 2866m, 2930m, 2952m.

### Exemple 9 : Composition parfumante contenant le composé 5

Un accord de type bonbon au citron a été préparé à partir des ingrédients suivants

| **Ingrédients** | **Accord A** | **Accord B** |
|---|---|---|
| THYMOL 10%DPG | 5 | 5 |
| LABIENOXIME 10% DPG | 5 | 5 |
| CITRONELLYL ACETATE | 10 | 10 |
| ALCOOL C9 | 1 | 1 |
| HEXYL CINNAMIQUE ALDEHYDE | 50 | 50 |
| ALDEHYDE C8 | 5 | 5 |
| ALDEHYDE C9 | 2 | 2 |
| PINENE BETA | 50 | 50 |
| CITRONELLOL 98% | 10 | 10 |
| GALAXOLIDE SANS PE DANS MIP | 100 | 100 |
| M.C.P HYDRATEE NATURELLE | 1 | 1 |
| TRIPLAL | 2 | 2 |
| ETHYL MALTOL | 2 | 2 |
| VERTENEX N2 | 30 | 30 |
| CITRON ESS Italie SS CITROPTENE | 300 | 300 |
| LEMONILE | 30 | 30 |
| DPG | 397 | 297 |
| **Composé 5** | **0** | **100** |

Evaluation : L'ajout du composé **5** donne beaucoup de puissance à cette note citron, en boostant considérablement les notes "citron" et "nitrile". La molécule a également une action sur la facette gourmande, la note éthyle maltol est accentuée. Le même effet est constaté dans l'application alcoolique et shampooing.

### Exemple 10 : Composition parfumante contenant le composé 5

Un accord de type pêche verte a été préparé à partir des ingrédients suivants :

| **Ingrédients** | **Accord A** | **Accord B** |
|---|---|---|
| CORPS X24242 1%DPG | 20 | 20 |
| MELONAL M13303 | 1 | 1 |
| BUCHU FEUILLE SS | 1 | 1 |
| ETHYLE ACETATE | 80 | 80 |
| ETHYLE ACETYL ACETATE | 50 | 50 |
| PHENYLETHYLIQUE ALCOOL | 50 | 50 |
| BENZOIQUE ALDEHYDE | 5 | 5 |
| ALDEHYDE C14 | 40 | 40 |
| ALDEHYDE C19 | 20 | 20 |
| DELTA DODECALACTONE | 10 | 10 |
| DAMASCENONE | 1 | 1 |
| DELTA DECALACTONE | 15 | 15 |
| GAMMA DODECALACTONE | 10 | 10 |
| GAMMA DECALACTONE | 80 | 80 |
| GAMMA VALEROLACTONE | 15 | 15 |
| HEXENOL CIS 3 | 80 | 80 |
| IONONE ALPHA | 4 | 4 |
| JASMONE CIS | 4 | 4 |
| LINALOL | 150 | 150 |
| MUSC T | 150 | 150 |
| HEXENYL CIS 3 SALICYLATE | 80 | 80 |
| NECTARYL | 15 | 15 |
| DPG | 109 | 49 |
| **Composé 5** | **0** | **60** |

Evaluation : L'ajout du composé **5** augmente considérablement la puissance, elle pousse les notes vertes. Plusieurs personnes d'un panel entraîné ont la même opinion : ils préfèrent la composition avec le composé **5.** Cette composition est à sentir en application shampooing.

## Revendications

1. Composé de formule (I) : 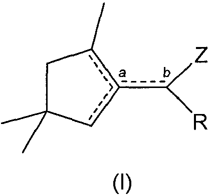 dans laquelle :
R est un atome d'hydrogène, un groupe alkyle en C₁-C₆, un groupe alcényle en C₂-C₆ ou un groupe =CH₂ ;
Z est un groupe CN ou CHO ; et
au plus une des liaisons en pointillé est présente ;
à condition que :
si Z est un groupe CHO et une des liaisons en pointillé est présente, alors R n'est pas un atome d'hydrogène, et
si la liaison en pointillé entre les carbones Ca et Cb est présente, alors R n'est pas un groupe =CH₂.

2. Composé selon la revendication 1 ayant pour formule générale (I') : 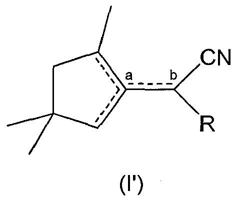 dans laquelle :
R est un atome d'hydrogène, un groupe alkyle en C₁-C₆, alcényle en C₂-C₆ ou un groupe =CH₂ ; et
au plus une des liaisons en pointillé est présente ;
à condition que :
si la liaison en pointillé entre les carbones Ca et Cb est présente, alors R n'est pas un groupe =CH₂.

3. Composé selon la revendication 2, dans lequel R est un atome d'hydrogène ou un groupe méthyle ou =CH₂.

4. Composé selon la revendication 1 ayant pour formule générale (I") : 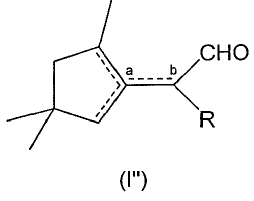 dans laquelle :
R est un atome d'hydrogène, un groupe alkyle en C₁-C₆, alcényle en C₂-C₆ ou un groupe =CH₂ ; et
au plus une des liaisons en pointillé est présente ;
à condition que :
si une des liaisons en pointillé est présente, alors R n'est pas un atome d'hydrogène, et
si la liaison en pointillé entre les carbones Ca et Cb est présente, alors R n'est pas un groupe =CH₂.

5. Composé selon la revendication 4, dans lequel R est un atome d'hydrogène, groupe méthyle ou un groupe =CH₂.

6. Procédé de préparation d'un composé de formule (I) **caractérisé en ce que** ledit procédé comprend une étape consistant à faire réagir le composé de formule (A) 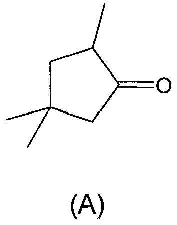 avec un diéthylphosphonodialkylacétonitrile de formule RCH(CN)PO(OC₂H₅)₂ dans laquelle R est un atome d'hydrogène, un groupe alkyle en C₁-C₆ ou un groupe alcényle en C₂-C₆ ; ou
avec un cyanoacétate d'alkyl de formule NCCH₂COOR' dans laquelle R' est un groupe alkyl en C₁-C₄.

7. Utilisation d'un composé selon l'une quelconque des revendications 1 à 5 en tant qu'agent ou composé fragrant.

8. Utilisation d'un composé selon l'une quelconque des revendications 1 à 5 en tant qu'agent masquant d'une odeur.

9. Utilisation selon l'une quelconque des revendications 7 et 8, dans laquelle le composé selon l'une quelconque des revendications 1 à 5 est en combinaison avec au moins un autre ingrédient aromatisant ou parfumant, et/ou au moins un solvant, et/ou au moins un adjuvant.

10. Composition parfumante **caractérisée en ce qu'**elle comprend un composé selon l'une quelconque des revendications 1 à 5.

## Patentansprüche

1. Verbindung der Formel (I): 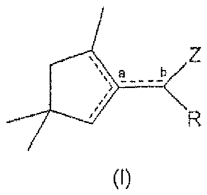 in der:
R ein Wasserstoffatom, eine C₁-C₆-Alkylgruppe, eine C₂-C₆-Alkenylgruppe oder eine Gruppe =CH₂ ist;
Z eine Gruppe CN oder CHO ist und
höchstens eine der gestrichelten Bindungen vorhanden ist;
mit der Maßgabe, dass:
wenn Z eine Gruppe CHO ist und eine der gestrichelten Bindungen vorliegt, dann R kein Wasserstoffatom ist, und
wenn die gestrichelte Bindung zwischen den Kohlenstoffen Ca und Cb vorliegt, dann R keine Gruppe =CH₂ ist.

2. Verbindung gemäß Anspruch 1, die die allgemeine Formel (I') hat: 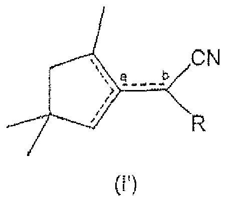 in der:
R ein Wasserstoffatom, eine C₁-C₆-Alkylgruppe, eine C₂-C₆-Alkenylgruppe oder eine Gruppe =CH₂ ist und
höchstens eine der gestrichelten Bindungen vorhanden ist;
mit der Maßgabe, dass:
wenn die gestrichelte Bindung zwischen den Kohlenstoffen Ca und Cb vorhanden ist, dann R keine Gruppe =CH₂ ist.

3. Verbindung gemäß Anspruch 2, in der R ein Wasserstoffatom oder eine Methylgruppe oder =CH₂ ist.

4. Verbindung gemäß Anspruch 1, die die allgemeine Formel (I") hat: 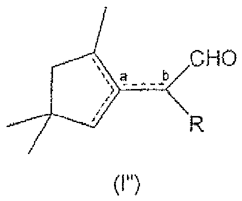 in der:
R ein Wasserstoffatom, eine C₁-C₆-Alkylgruppe, eine C₂-C₆-Alkenylgruppe oder eine Gruppe =CH₂ ist und
höchstens eine der gestrichelten Bindungen vorhanden ist;
mit der Maßgabe, dass:
wenn eine der gestrichelten Bindungen vorhanden ist, dann R kein Wasserstoffatom ist, und
wenn die gestrichelte Bindung zwischen den Kohlenstoffen Ca und Cb vorhanden ist, dann R keine Gruppe =CH₂ ist.

5. Verbindung gemäß Anspruch 4, in der R ein Wasserstoffatom, eine Methylgruppe oder eine Gruppe =CH₂ ist.

6. Verfahren zur Herstellung einer Verbindung der Formel (I), **dadurch gekennzeichnet , dass** das Verfahren eine Stufe umfasst, die darin besteht, die Verbindung der Flormel (A) 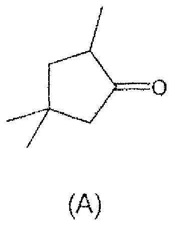 mit einem Diethylphosphonodialkylacetonitril der Formel RCH(CN)PO(OC₂H₅)₂, in der R ein Wasserstoffatom, eine C₁-C₆-Alkylgruppe oder eine C₂-C₆-Alkenylgruppe ist, oder
mit einem Alkylcyanoacetat der Formel NCCH₂CCOR', in der R' eine C₁-C₄-Alkylgruppe ist, reagieren zu lassen.

7. Verwendung einer Verbindung gemäß einem der Ansprüche 1 bis 5 als Duftmittel oder Duftverbindung.

8. Verwendung einer Verbindung gemäß einem der Ansprüche 1 bis 5 als Maskierungsmittel für einen Geruch.

9. Verwendung gemäß einem der Ansprüche 7 und 8, wobei die Verbindung gemäß einem der Ansprüche 1 bis 5 in Kombination mit wenigstens einem anderen aromatisierenden oder parfümierenden Ingrediens und/oder wenigstens einem Lösungsmittel und/oder wenigstens einem Adjuvans ist.

10. Parfümierende Zusammensetzung, **dadurch gekennzeichnet, dass** sie eine Verbindung gemäß einem der Ansprüche 1 bis 5 umfasst.

## Claims

1. A compound of formula (I): 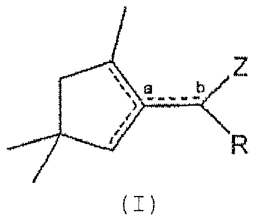 in which:
R is a hydrogen atom, a C₁-C₆ alkyl group, a C₂-C₆ alkenyl group or a =CH₂ group;
Z is a CN or CHO group; and
at most one of the bonds represented by dashes is present;
provided that:
if Z is a CHO group and one of the bonds represented by dashes is present, then R is not a hydrogen atom, and if the bond represented by dashes between the carbons Ca and Cb is present, then R is not a =CH₂ group.

2. A compound according to claim 1 having the general formula (I'): 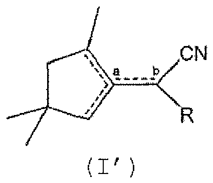 in which:
R is a hydrogen atom, a C₁-C₆ alkyl group, a C₂-C₆ alkenyl group or a =CH₂ group; and
at most one of the bonds represented by dashes is present;
provided that:
if the bond represented by dashes between the carbons Ca and Cb is present, then R is not a =CH₂ group.

3. A compound according to claim 2, in which R is a hydrogen atom or a methyl or =CH₂ group.

4. A compound according to claim 1 having the general formula (I") : 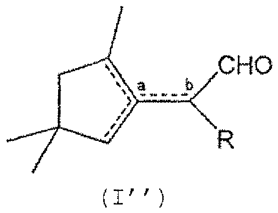 in which:
R is a hydrogen atom, a C₁-C₆ alkyl group, a C₂-C₆ alkenyl group or a =CH₂ group; and
at most one of the bonds represented by dashes is present;
provided that:
if one of the bonds represented by dashes is present, then R is not a hydrogen atom, and
if the bond represented by dashes between the carbons Ca and Cb is present, then R is not a =CH₂ group.

5. A compound according to claim 4, in which R is a hydrogen atom or a methyl or =CH₂ group.

6. A process for the preparation of a compound of formula (I), **characterised in that** the process comprises a step that consists in reacting the compound of formula (A): 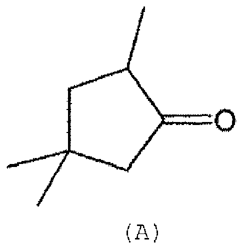 with a diethylphosphonodialkylacetonitrile of formula RCH(CN)PO(OC₂H₅)₂ in which R is a hydrogen atom, a C₁-C₆ alkyl group or a C₂-C₆ alkenyl group; or
with an alkyl cyanoacetate of formula NCCH₂COOR' in which R' is a C₁-C₄ alkyl group.

7. Use of a compound according to any one of claims 1 to 5 as a fragrant agent or compound.

8. Use of a compound according to any one of claims 1 to 5 as a masking agent for an odour.

9. Use according to any one of claims 7 and 8, in which the compound according to any one of claims 1 to 5 is in combination with at least one other aromatizing or scenting ingredient, and/or at least one solvent, and/or at least one adjuvant.

10. A scenting composition, **characterised in that** it comprises a compound according to any one of claims 1 to 5.
